# EUROPEAN PATENT SPECIFICATION

(11) **EP 1 674 059 B1**
(45) Date of publication and mention of the grant of the patent: **20.08.2008**
(21) Application number: 04030644.1
(22) Date of filing: 23.12.2004
(51) Int. Cl.: A61F 11/08

(54) **Hearing protection earplug and use of the same**
Gehörschutzstöpsel und dessen Verwendung
Bouchon de protection auditive et son utilisation

(43) Date of publication of application: 28.06.2006
(73) Proprietor: PHONAK AG, 8712 Stäfa (CH)
(72) Inventor: Kurth, Roland, 2520 La Neuveville (CH)
(74) Representative: Schwan - Schwan - Schorer

(56) References cited:
- EP-A- 1 071 307
- WO-A-02/50499

## Description

The invention relates to a hearing protection earplug according to the preamble of claim 1 and to a method for manufacturing such an earplug.

US 2003/0112990 A1 relates to a hearing protection earplug comprising a customized shell with an outer shape adapted to the inner shape of the user's outer ear and ear canal. The shell comprises a sound passage extending through the earplug, with the outer end of the sound passage being provided with a slit membrane which closes the sound passage whenever engaged by a remote instrument such as a microphone of a measurement device. The shell further comprises a receptacle for an insert member which may be a communication element, i.e. an active unit, comprising a microphone and a speaker, and which may be releasably engaged within the receptacle of the shell. The receptacle communicates with a second sound passage extending through the earplug.

US 2002/0080979 A1 relates to a hearing protection earplug comprising a soft shell, i.e. a shell made of a relatively resilient material which is capable of adapting its outer shape to the inner shape of the user's outer ear and ear canal, into which soft shell an electronic module, i.e. an active unit, may be inserted in a detachable manner for enabling exchange of the soft shell. The electronic module comprises a microphone, a signal processing unit and a speaker for providing for an active hearing protection function.

US 5,631,965 relates to a hearing protection earplug comprising a soft shell and an active unit which is screwed into the shell. The active unit includes a microphone, a signal processing unit and a speaker, with the speaker communicating with a sound passage extending through the shell.

US 6,687,377 B2 relates to a hearing protection earplug comprising a shell with a sound measurement channel which extends through the shell and which terminates at an opening at the outer end of the shell. A remote device such as a sound measurement device may be temporarily inserted into the outer opening of the sound measurement passage.

US 2003/0037989 A1 relates to an earplug comprising a customized shell which is provided with a receptacle into which a hearing aid module comprising a microphone, a signal processing unit and a speaker may be releasably inserted for allowing exchange of the shell.

The speaker of the hearing aid module communicates with a sound passage extending through the shell.

EP 1 071 307 A1 relates to an earplug according to the preamble of claim 1, wherein the active unit can be fixed in a detachable manner in a moulded cavity of the shell and wherein the active unit, after having been removed from the moulded cavity can be replaced by a sealing cap which can be fixed in a detachable manner in the moulded cavity.

It is an object of the invention to provide for a hearing protection earplug which allows for a highly flexible use by enabling the user to select between different functions in a simple and easy manner. It is a further object of the invention to provide for a manufacturing method and a use of such an earplug.

The above objects are achieved by a hearing protection earplug as defined in claim 1, by a use of such a hearing protection earplug as defined in claim 26, by a method for manufacturing such an earplug as defined in claim 29.

The invention is beneficial in that the hearing protection earplug can be used, according to the desires of the user, either without the active unit being inserted into the shell, with the hearing protection earplug in this case acting as a passive hearing protection earplug with the sound passage being closed by the valve means, or with the active unit being inserted into the shell, with the hearing protection earplug in this case being provided with additional functionality, such as a selective communication function, i.e. the earplug acting as an active hearing protection device, or an in-situ sound attenuation measurement function, with the active unit being acoustically connected via the opened valve means with the sound passage. The invention also allows for alternatively using active units having different functionality with the same earplug in order to provide for a particularly high flexibility of use. Due to the provision of the acoustic valve means the change between different use modes and hence the handling of the earplug by the user is particularly simple. Further, due to the fact that the active unit can be easily removed from the shell, the earplug is easy to clean. Finally, by manufacturing the shell together with the valve means by an additive layer-by-layer build-up process, the valve means can be implemented in a particularly simple manner without the need of a separate assembly step for mounting the valve means at the shell.

Preferred embodiments of the invention are defined in the dependent claims.

In the following, examples of the invention will be illustrated by reference to the attached drawings, wherein:
- Fig. 1a and 1b: show a schematic side view, partially in cross-section, of the distal end of a first embodiment of a hearing protection earplug according to the invention, wherein the active unit is shown in a condition prior to being fixed within the shell and after having been fixed within the shell, respectively; and
- Fig. 2 to Fig. 4: show views, similar to that of Fig. 1a, of modified embodiments of the invention.

The present invention relates to hearing protection earplugs comprising a shell which is adapted to be worn at least in part in a user's ear canal, i.e. at least a distal portion of the shell is to be inserted into the outer part of the user's ear canal in order to provide for an acoustic attenuation of at least 10 dB averaged over the audible frequency range when the earplug is worn by the user, in order to protect the user from excessive levels of ambient sound. The earplug may comprise an acoustic filter for adjusting the desired total acoustic attenuation or for adjusting the frequency dependent acoustic attenuation.

The shell preferably is a hard shell having an elasticity from shore D85 to D65 and preferably is made of polyamide. In order to achieve optimized fit of the shell within the user's outer ear and ear canal, the shell preferably has an outer surface individually shaped according to the measured shape of the user's outer ear and ear canal, i.e. the shell preferably has an individually customized outer shape. The shape of the user's outer ear and ear canal may be determined by direct three-dimensional scanning of the ear canal and the concha or by producing an impression of the ear canal and the concha which subsequently undergoes scanning. The scanning process may be carried out optically, preferably by laser scanning.

The digital data obtained by the scanning process is then used to create the hard shell by an additive or incremental layer-by-layer build up process. Such processes are also known as "rapid prototyping". A preferred additive build-up process is a layer-by-layer laser sintering process of powder material, preferably polyamide powder. Such processes are also known as "selective laser sintering" (SLS). The basic principle therein is the repeated deposition of a thin layer of material on a surface, with the desired sectional shape then being stabilized, i.e. hardened, by laser action. An overview regarding such processes can be found, for example, in US 2003/0133583 A1 or US 6,533,062 B1.

According to the invention, the shell is provided with a sound passage which extends between an outer opening of the shell and an inner sound output opening at the distal end of the shell. Further, the shell comprises valve means which are movable between a closed position in which the valve means acoustically open the sound passage, with the valve means being biased towards the closed position. The shell further is adapted to be detachably connected with an active unit which causes the valve means to move from the closed position into the open position upon engagement between the active unit and the shell. Thereby it is possible to use the earplug as a passive hearing protection earplug with the active unit being removed, or as a hearing protection earplug with added functionality provided by the active unit when connected to the shell.

Preferably, the valve means provides in the closed position for an acoustic attenuation of at least 10 dB averaged over the audible frequency range. In general, the overall acoustic attenuation provided by the earplug should be at least 10 dB more when the valve means are closed, compared to the case when the valve means are open.

The active unit may comprise an acoustic output transducer and means for producing audio signals for the output transducer, with the output transducer being acoustically connected to the sound output opening of the shell via the sound passage when the active unit is connected to the shell. The means for producing audio signals for the output transducer may comprise a microphone included within the active unit for sensing ambient sound and/or an interface for wireless connection with a remote audio signal source, such as a remote microphone. In both cases the active unit provides for an active hearing protection function to the earplug by enabling communication, in particular speech communication, via the microphone and the output transducer while the earplug is worn by the user.

Alternatively or in addition, the active unit may comprise a microphone which is adapted to be acoustically connected to the sound output opening of the shell via the sound passage when the active unit is connected to the shell. In this case, the microphone may be used for in-situ attenuation measurements of the earplug when worn by the user. In addition, the active unit may comprise dosimeter means connected to the microphone for determining the actual sound exposure experienced by the user's ear. The dosimeter means may be adapted for comparing the determined actual sound exposure to regulations implemented in the dosimeter means in order to judge whether the sound exposure complies with the regulations.

Usually the active unit comprises an audio signal processing unit for processing input audio signals provided by the microphone sensing ambient sound into the output audio signals for the output transducer. If a remote microphone is provided, the interface for wireless connection may be a radio frequency interface, in particular a frequency modulated radio signal interface or a Bluetooth interface, an inductive interface or an infrared interface.

In general, the earplug may be designed such that the user not only has the choice to use the earplug with or without the active unit but in addition the user has the option to select between functionally different active units, for example, one with a microphone and an output transducer for providing for an active hearing protection function, and one with a microphone and dosimeter means for providing for a dosimeter and sound exposure monitoring function. Thereby the flexibility of the hearing protection system is further enhanced.

In general, it is possible and desirable to manufacture not only the shell by a layer-by-layer build-up process, but also the functional components integrated within the shell, such as the valve means, whereby the manufacturing process can be significantly simplified.

In the following, examples of the design of the means for connecting the active unit to the shell and the valve means will be illustrated by reference to the drawings.

In general, the connecting means may be adapted for clipping, screwing or bayonet coupling the active unit to the shell. However, also any other quickly detachable connection may be used.

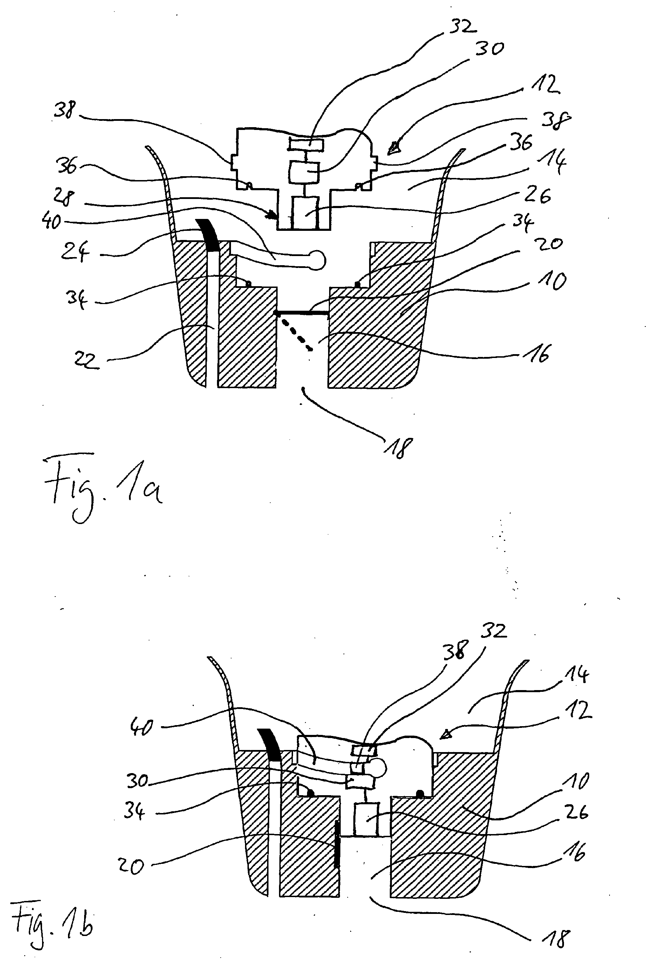

Figs. 1a and 1b show an example where an active unit 12 is connected to a shell 10 by a bayonet coupling mechanism. In all Figures, only the distal portions of the shell 10 and the active unit 12 are shown, with the distal end of the shell being located at the bottom of each Figure. The shell 10 comprises an outer cavity 14 forming an outer opening into which the active unit 12 can be inserted for being connected to the shell 10. The outer cavity 14 is connected at its distal end to a sound passage 16 which extends between the outer cavity 14 and an inner sound output opening 18 provided at the distal end of the shell 10.

Within the sound passage 16 a valve member 20 is provided which is designed as a lid which is adapted to be tilted from a closed position, in which the lid 20 extends over the entire cross-section of the sound passage 16 (see Fig. 1a), into an open position in which the lid 20 exposes the sound passage 20 (see Fig. 1b). The dashed lines in Fig. 1a indicate an intermediate position of the lid 20. In the closed position, the lid 20 serves to acoustically close the sound passage 16 regarding the outer cavity 14.

In addition to the sound passage 16 the shell 10 may comprise a sound channel 22 extending from the outer cavity 14 to the distal end of the shell 10 which is acoustically closed by a passive filter element 24. The filter element 24 is provided for achieving a defined frequency dependent acoustic attenuation by the shell 10, whereby, for example, speech frequencies may be attenuated less than high frequency noise.

The active unit 12 comprises an acoustic output transducer 26, i.e. a speaker, at its distal end, which distal end is formed as an axial protection 28 adapted to move the lid 20 from the closed position shown in Fig. 1a into the open position shown in Fig. 1b, when the active unit 12 is connected to the shell 10. The lid 20 is biased towards the closed position of Fig. 1a in order to acoustically close the sound passage 16 when the active unit 12 is removed from the shell 10. In the position of Fig. 1b, wherein the active unit 12 is connected to the shell 10, the output transducer 26 is acoustically connected to the inner sound outlet opening 18 via the open sound passage 16.

The active unit 12 further comprises an audio signal processing unit 30 which processes input audio signals received from a microphone 32 into output audio signals for the output transducer 26.

The shell 10 is provided with a sealing lip 34 adapted for engagement with a mating groove 36 at the active unit 12 in order to seal the outer end of the sound passage 16 regarding the outer cavity 14 and hence regarding the environment around the user when the active unit 12 is connected to the shell 10.

The active unit 12 further comprises radial projections 38 which are adapted for engagement with mating slots 40 provided at the shell 10. The slots 40 and the radial projections 38 are designed for providing for a bayonet-like engagement between the active unit 12 and the shell 10 when the active unit 12 is inserted into the shell 10 and is slightly rotated against the shell 10.

When the active unit 12 is removed from the shell 10 again the lid 10 will automatically move into the closed position shown in Fig. 1a due to the biasing forces.

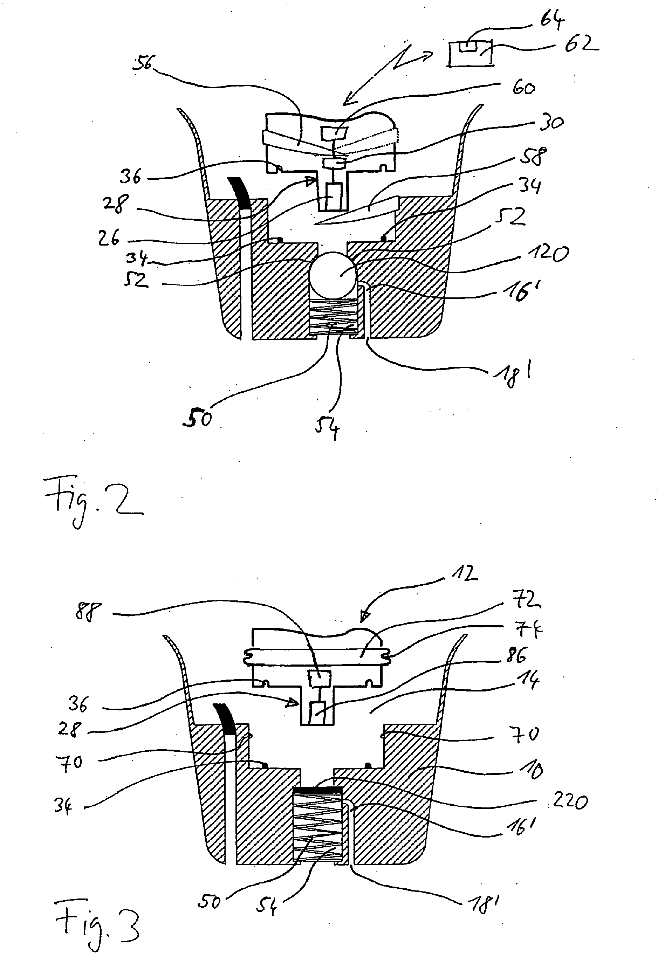

In Fig. 2 a modified embodiment is shown in which the bayonet mechanism of Fig. 1a and 1b is replaced by a screw mechanism and wherein the lid-like valve member 20 is replaced by a ball-like valve member 120.

In general, Fig. 2 is an example of the case in which the acoustic valve comprises a valve member which is axially movable within the sound passage between the closed position and the open position, with the valve member being biased towards the closed position by a spring element which is arranged distal from the valve member.

In the embodiment shown in Fig. 2, a spring element 50 rests against the distal end of the shell 10 in order to bias the ball 120 outwardly against a spherically shaped surface 52 of the sound passage 16 in order to acoustically close the sound passage 16 when the active 12 is not connected to the shell 10. The spring element 50 is located within an inner cavity 54 of the shell. In the embodiment of Fig. 2 the sound passage 16 comprises a portion 16' which is angled regarding the axial direction of the shell 10 and which is located distal from the closed position of the ball 120. In the embodiment of Fig. 2 the angled portion 16' of the sound passage 16 extends to an outer sound outlet opening 18' at the distal end of the shell 10, with the angled portion 16' thus bypassing the inner cavity 54 in which the spring element 50 is located.

When the active unit 12 is inserted into the shell 10, the axial protection 28 will axially move the ball 120 from the closed position of Fig. 2 towards the distal end of the shell 10 against the biasing force of the spring element 50, thereby reaching the open position in which the centre of the ball 120 is located distal from the point where the angled portion 16' starts, thereby acoustically opening the sound passage 16, 16' extending from the outer cavity 14 towards the inner sound outlet opening 18'.

In the embodiment of Fig. 2, the active unit 12 and the shell 10 are provided with mating threads 56, 58 in order to allow the active unit 12 to be screwed into the shell 10 for connecting the active unit 12 to the shell 10. Preferably the thread is so steep that rotation corresponding to from a half turn to a full turn is sufficient for achieving engagement between the active unit 12 and the shell 10.

A further difference between the embodiment of Fig. 1a and 1b and Fig. 2 is that in the embodiment of Fig. 2 the internal microphone 32 of the active unit 12 is replaced by an interface 60 adapted for wireless communication with a remote audio signal source 62. The remote audio signal source 62 preferably comprises a microphone 64 in order to allow for wireless communication between the person using the earplug comprising the shell 10 and the active unit 12 and a second person using the external audio source 62 with the external microphone 64. The interface 60 may be, for example, a frequency modulated radio frequency interface or a Bluetooth interface.

In the embodiment of Fig. 3 the ball 120 is replaced by a valve plate 220 which is located in the closed position proximal from the point where the angled portion 16' of the sound passage 16 starts, while the plate 220 is located in the open position proximal from that point in order to open the sound passage 16, 16' from the outer cavity 14 to the inner sound outlet opening 18'. In the closed position the valve plate 220 is biased by the spring element 50 against an axially oriented surface of the sound passage 16.

Further, the embodiment of Fig. 3 is an example for a clipping or snap-in mechanism for connecting the active unit 12 with the shell 10. Such mechanism may be generally achieved by providing either the active unit 12 or the shell 10 with a radially movable or resilient element adapted to engage a mating element provided at the counterpart, i.e. the shell 10 or the active unit 12, respectively.

More specifically, the embodiment of Fig. 3 is an example in which the shell 10 is provided with a rigid radially projecting projection 70, while the active unit 12 is provided with a soft lip 72 comprising a circumferential groove 74 into which the projections 70 of the shell 10 will engage when the active unit is axially pressed into the outer cavity 14 of the shell 10.

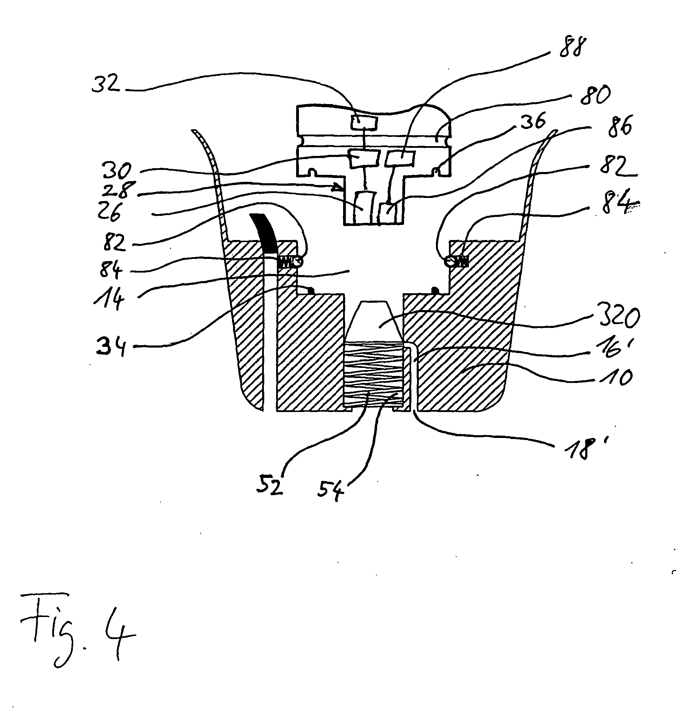

In the embodiment of Fig. 4 a modification of the clipping/snap-in mechanism is shown wherein the resilient lip 72 is replaced by a circumferential groove 80 provided at the outer surface of the active 12 and wherein the rigid projections 70 of the shell 10 are replaced by radially movable elements 82 which are radially biased towards the centre of the shell 10 by spring elements 84. In the example shown in Fig. 4 the engagement elements 84 are balls.

Further, in the embodiment of Fig. 4 the valve plate 220 is replaced by a conical body 320 which is tapered towards its outer end while its distal end fills the entire cross-section of the sound passage 16. In the closed position shown in Fig. 4 the distal end of the valve body 320 is located proximal from the point where the angled portion 16' of the sound passage 16 starts, while in the open position the valve body 320 is forced into the distal direction by the axial projection 28 of the active unit 12 so far that the distal end of the valve body 320 is located distal from the point where the angled portion 16' of the sound passage 16 starts, so that an acoustic connection between the outer cavity 14 and the inner sound outlet opening 18' via the sound passage 16, 16' is opened.

In the embodiment of Fig. 4 the active unit is provided with a distal microphone 86 which may be present alternatively or in addition to an output transducer 26. The distal microphone 86 may serve for performing in-situ sound attenuation measurements when the shell 10 is worn within the user's ear canal. Alternatively or in addition the distal microphone 86 may be connected to a digital data processing unit 88 which may serve as a dosimeter for determining and recording the actual sound exposure experienced by the user when wearing the earplug. In addition, the unit 88 may serve to compare the determined sound exposure to regulations implemented in the unit 88 in order to judge whether the determined sound exposure complies with the regulations, whereby a corresponding alarm signal may be output, i.e. an alarm sound or synthetic speech, if the regulations are infringed.

In Fig. 3 an example of an active unit 12 is shown which includes only a distal microphone 86 and a digital data processing unit 88 serving as a dosimeter.

## Claims

1. A hearing protection earplug comprising a shell (10) to be worn at least in part in a user's ear canal, said shell being adapted to provide for a mechanical acoustic attenuation of at least 10 dB averaged over the audible frequency range when worn by the user, an active unit (12) comprising an acoustic output transducer (26) and means (30, 32, 60) for producing output audio signals for said output transducer and/or comprising a microphone (86), and means (34, 36, 38, 40, 56, 58, 80, 82, 84) for detachably connecting said active unit to said shell, wherein said shell comprises a sound passage (16, 16') extending between an outer opening (14) of said shell and an inner sound output opening (18, 18') at the distal end of said shell, **characterized by** said shell comprising valve means (20, 120, 320) which are moveable, upon connecting said active unit to said shell, from a closed position in which said valve means acoustically close said sound passage into an open position in which said valve means acoustically open said sound passage, said valve means being biased towards said closed position in order to acoustically close said sound passage when said active unit is removed from said shell, and wherein said output transducer and/or said microphone is acoustically connected to said sound output opening via said sound passage when said active unit is connected to said shell.

2. The earplug of claim 1, wherein said connecting means (34, 36, 38, 40, 56, 58, 70., 72, 74, 80, 82, 84) comprise sealing means (34, 36) for sealing the outer end of said sound passage (16, 16') regarding the environment around said user by engagement with said active unit (12).

3. The earplug of one of claims 1 and 2, wherein said connecting means (34, 36, 38, 40, 56, 58, 70., 72, 74, 80, 82, 84) are adapted for clipping, screwing or bayonet coupling said active unit (12) to said shell (10).

4. The earplug of one of claims 1 and 2, wherein said connecting means (34, 36, 70, 72, 74, 80, 82, 84) are adapted for clipping said active unit (12) to said shell (10) and wherein one of said active unit and said shell comprises a radially movable (82) or resilient element (72) adapted to engage a mating element (70, 80) provided at the other one of said active unit and said shell.

5. The earplug of claim 4, wherein said radially movable element (82) is biased radially towards the center of said shell (10).

6. The earplug of one of the preceding claims, wherein said shell (10) comprises an outer cavity (14) into which said active unit (12) is to be inserted.

7. The earplug of one of the preceding claims, wherein said valve means (20, 120, 220, 320) are adapted to provide in said closed position for an acoustic attenuation of at least 10 dB averaged over the audible frequency range.

8. The earplug of one of the preceding claims, wherein said valve means comprise a lid (20) which is adapted to be tilted, upon connecting said active unit (12) to said shell (10), from said closed position in which said lid extends over the entire cross section of said sound passage (16) into said open position in which said lid exposes said sound passage.

9. The earplug of one of the preceding claims, wherein said active unit (12) comprises a projection (28) for moving said valve means (20, 120, 220, 320), upon connecting said active unit to said shell (10), from said closed position to said open position.

10. The earplug of claim 9, wherein said valve means comprise a valve member (120, 220, 320) which is axially movable within said sound passage (16) by said projection (28) between a first axial position in which said valve member acoustically closes said sound passage and a second position in which said valve member acoustically opens said sound passage.

11. The earplug of claim 10, wherein said valve member (120, 220, 320) is biased towards said first axial position by a spring element (52) arranged at least in part within said sound passage (16).

12. The earplug of claim 11, wherein said spring element (52) is arranged distal from said valve member (120, 220, 320).

13. The earplug of one of claims 10 to 12, wherein said valve member (120, 220) rests in said first axial position against a surface (52) of said sound passage (16) for acoustically closing said sound passage.

14. The earplug of one of claims 10 to 13, wherein said valve member is a ball (120) or a plate (220).

15. The earplug of one of claims 10 to 14, wherein said sound passage (16, 16') comprises a portion (16') which is angled regarding the axial direction of said shell (10) and which is located distal from said first position of said valve member (120, 220, 320).

16. The earplug of claim 1, wherein said means for producing audio output signals comprises a microphone (32) for converting ambient sound into input audio signals and an audio signal processing unit (30) for processing said input audio signals into said output audio signals.

17. The earplug of claim 1, wherein said means for producing audio output signals comprise an interface (60) for wireless connection with a remote audio signal source such as a remote microphone (64).

18. The earplug of claim 17, wherein said interface (60) is a radio frequency interface, an inductive interface or an infrared interface.

19. The earplug of claim 18, wherein said interface (60) is a Bluetooth interface.

20. The earplug of one of claims 1 or 16, wherein said active unit (12) comprises dosimeter means (88) for determining, by said microphone (86), the sound exposure of said user.

21. The earplug of claim 20, wherein said dosimeter means (88) are adapted for comparing said determined sound exposure to regulations implemented in said dosimeter means in order to judge whether said sound exposure complies with said regulations.

22. The earplug of one of the preceding claims, wherein said shell (10) has an outer surface individually shaped according to the measured inner shape of the user's outer ear and ear canal.

23. The earplug of claim 22, wherein said shell (10) has an elasticity of from shore D85 to shore D65.

24. The earplug of claim 23, wherein said shell (10) is made of polyamide.

25. The earplug of claim 18, wherein said interface (60) is a frequency modulated radio signal interface.

26. A use of a hearing protection earplug according to one of the preceding claims, comprising:
connecting said active unit (12) to said shell (10) by engaging said connecting means (34, 36, 38, 40, 56, 58, 70., 72, 74, 80, 82, 84), inserting said earplug at least in part into said user's ear canal, removing said earplug from said user's ear canal, disconnecting said active unit from said shell by disengaging said connecting means, and inserting said earplug at least in part into said user's ear canal.

27. The use of claim 26, further comprising: removing said earplug from said user's ear canal, connecting a further active unit (12) which is functionally different from said active unit (12) to said shell (10) by engaging said connecting means (34, 36, 38, 40, 56, 58, 70., 72, 74, 80, 82, 84), and inserting said earplug at least in part into said user's ear canal.

28. A hearing protection system comprising an earplug of one of claims 1 to 25, further comprising a plurality of functionally different active units (12) comprising an acoustic output transducer (26) and means (30, 32, 60) for producing output audio signals for said output transducer and/or a microphone (86), wherein said connecting means (34, 36, 38, 40, 56, 58, 70., 72, 74, 80, 82, 84) are adapted for detachably connecting a selected one of said active units to said shell, and wherein valve means (20, 120, 220, 320) are moveable, upon connecting said selected one of said active units to said shell, from a closed position in which said valve means acoustically close said sound passage into an open position in which said valve means acoustically open said sound passage, said valve means being biased towards said closed position in order to acoustically close said sound passage when said selected one of said active units is removed from said shell, and wherein said output transducer and/or said microphone is acoustically connected to said sound output opening via said sound passage when said selected one of said active units is connected to said shell.

29. Method for manufacturing a hearing protection earplug comprising:
providing a shell (10) to be worn at least in part in a user's ear canal, said shell being adapted to provide for a mechanical acoustic attenuation of at least 10 dB averaged over the audible frequency range when worn by the user, and said shell comprising a sound passage (16, 16') extending between an outer opening (14) of said shell and an inner sound output opening (18, 18') at the distal end of said shell; and
providing an active unit (12) comprising an acoustic output transducer (26) and means (30, 32, 60) for producing output audio signals for said output transducer and/or a microphone (86), said active unit being adapted for being detachably connected to said shell;
said shell comprising valve means (20, 120, 220, 320) which are moveable, upon connecting said active unit to said shell, from a closed position in which said valve means acoustically close said sound passage into an open position in which said valve means acoustically open said sound passage, said valve means being biased towards said closed position in order to acoustically close said sound passage when said active unit is removed from said shell, and said output transducer and/or said microphone being acoustically connected to said sound output opening via said sound passage when said active unit is connected to said shell;
wherein said shell including said valve means is manufactured by an additive layer-by-layer build-up process.

## Patentansprüche

1. Gehörschutzstöpsel mit einer Schale (10), die mindestens zum Teil im Gehörgang eines Benutzers zu tragen ist und ausgebildet ist, um für eine mechanische akustische Dämpfung von mindestens 10 dB, gemittelt über den hörbaren Frequenzbereich, zu sorgen, wenn sie von dem Benutzer getragen wird, einer aktiven Einheit (12) mit einem akustischen Ausgangswandler (26) und Mitteln (30, 32, 60) zum Erzeugen eines Ausgangs-Audiosignals für den Ausgangswandler und/oder mit einem Mikrofon (86), sowie Mitteln (34, 36, 38, 40, 56, 58, 80, 82, 84) zum lösbaren Verbinden der aktiven Einheit mit der Schale, wobei die Schale einen Schalldurchlass (16, 16`) aufweist, der sich zwischen einer äußeren Öffnung (14) der Schale und einer inneren Schallauslassöffnung (18, 18') am distalen Ende der Schale erstreckt, **dadurch gekennzeichnet, dass** die Schale Ventilmittel (20, 120, 320) aufweist, die beim Verbinden der aktiven Einheit mit der Schale aus einer geschlossenen Stellung, in welcher die Ventilmittel den Schalldurchlass akustisch verschließen, in eine offene Stellung, in welcher die Ventilmittel den Schalldurchlass akustisch öffnen, verlagerbar ist, wobei die Ventilmittel zu der geschlossenen Stellung hin vorgespannt sind, um den Schalldurchlass akustisch zu schließen, wenn die aktive Einheit aus der Schale entfernt ist, und wobei der Ausgangswandler und/oder das Mikrofon über den Schalldurchlass akustisch mit der Schallauslassöffnung verbunden ist bzw. sind, wenn die aktive Einheit mit der Schale verbunden ist.

2. Ohrstöpsel gemäß Anspruch 1, wobei die Verbindungsmittel (34, 36, 38, 40, 56, 58, 70, 72, 74, 80, 82, 84) Abdichtmittel (34, 36) aufweisen, um das äußere Ende des Schalldurchlasses (16, 16`) mittels Eingriff mit der aktiven Einheit (12) bezüglich der den Benutzer umgebenden Umgebung abzudichten.

3. Ohrstöpsel gemäß Anspruch 1 oder 2, wobei die Verbindungsmittel (34, 36, 38, 40, 56, 58, 70, 72, 74, 80, 82, 84) ausgebildet sind, um die aktive Einheit (12) mit der Schale (10) mittels einer Clipsverbindung, Schraubverbindung oder Bajonett-Verbindung zu verbinden.

4. Ohrstöpsel gemäß Anspruch 1 oder 2, wobei die Verbindungsmittel (34, 36, 70, 72, 74, 80, 82, 84) ausgebildet sind, um die aktive Einheit (12) mit der Schale (10) zu verclipsen, und wobei entweder die aktive Einheit oder die Schale ein radial verlagerbares (82) oder nachgiebiges Element (72) aufweist, welches ausgebildet ist, um mit einem passenden Element (70, 80) Eingriff zu treten, das an dem jeweils anderen der Elemente aktive Einheit bzw. Schale ausgebildet ist.

5. Ohrstöpsel gemäß Anspruch 4, wobei das radial verlagerbare Element (82) radial zum Zentrum der Schale (10) hin vorgespannt ist.

6. Ohrstöpsel gemäß einem der vorhergehenden Ansprüche, wobei die Schale (10) einen äußeren Hohlraum (14) aufweist, in welchen die aktive Einheit (12) einzusetzen ist.

7. Ohrstöpsel gemäß einem der vorhergehenden Ansprüche, wobei die Ventilmittel (20, 120, 220, 320) ausgebildet sind, um in der geschlossenen Stellung für eine akustische Dämpfung von mindestens 10 dB, gemittelt über den hörbaren Frequenzbereich, zu sorgen.

8. Ohrstöpsel gemäß einem der vorhergehenden Ansprüche, wobei die Ventilmittel einen Deckel (20) aufweisen, der ausgebildet ist, um beim Verbinden der aktiven Einheit (12) mit der Schale (10) aus der geschlossenen Stellung, in welcher sich der Deckel über den gesamten Querschnitt des Schalldurchlasses (16) erstreckt, in die offene Stellung gekippt zu werden, in welcher der Deckel den Schalldurchlass freigibt.

9. Ohrstöpsel gemäß einem der vorhergehenden Ansprüche, wobei die aktive Einheit (12) einen Vorsprung (28) aufweist, um die Ventilmittel (20, 120, 220, 320) beim Verbinden der aktiven Einheit mit der Schale (10) aus der geschlossenen Stellung in die offene Stellung zu verlagern.

10. Ohrstöpsel gemäß Anspruch 9, wobei die Ventilmittel ein Ventilbauteil (120, 220, 320) aufweisen, welches innerhalb des Schalldurchlasses (16) mittels des Vorsprungs (28) zwischen einer ersten axialen Stellung, in welcher das Ventilbauteil den Schalldurchlass akustisch verschließt, und einer zweiten Stellung, in welcher das Ventilbauteil den Schalldurchlass akustisch öffnet, axial verlagerbar ist.

11. Ohrstöpsel gemäß Anspruch 10, wobei das Ventilbauteil (120, 220, 320) mittels eines Federelements (52), welches mindestens zum Teil innerhalb des Schalldurchlasses (16) angeordnet ist, zu der ersten axialen Stellung hin vorgespannt wird.

12. Ohrstöpsel gemäß Anspruch 11, wobei das Federelement (52) distal bezüglich des Ventilbauteils (120, 220, 320) angeordnet ist.

13. Ohrstöpsel gemäß einem der Ansprüche 10 bis 12, wobei das Ventilbauteil (120, 220) in der ersten axialen Stellung an einer Fläche (52) des Schalldurchlasses (16) anliegt, um den Schalldurchlass akustisch zu verschließen.

14. Ohrstöpsel gemäß einem der Ansprüche 10 bis 13, wobei es sich bei dem Ventilbauteil um eine Kugel (120) oder eine Platte (220) handelt.

15. Ohrstöpsel gemäß einem der Ansprüche 10 bis 14, wobei der Schalldurchlass (16, 16') einen Abschnitt (16') aufweist, der unter einem Winkel bezüglich der axialen Richtung der Schale (10) und distal bezüglich der ersten Stellung des Ventilbauteils (120, 220, 320) angeordnet ist.

16. Ohrstöpsel gemäß Anspruch 1, wobei die Mittel zum Erzeugen von Ausgangs-Audiosignalen ein Mikrofon (32) zum Umwandeln von Umgebungsschall in Eingangs-Audiosignale und eine Audiosignalverarbeitungseinheit (30) zum Verarbeiten der Eingangs-Audiosignale zu den Ausgangs-Audiosignalen aufweist.

17. Ohrstöpsel gemäß Anspruch 1, wobei die Mittel zum Erzeugen von Ausgangs-Audiosignalen eine Schnittstelle (60) zur drahtlosen Anbindung an eine externe Audiosignalquelle, wie beispielsweise ein externes Mikrofon (64), aufweisen.

18. Ohrstöpsel gemäß Anspruch 17, wobei es sich bei der Schnittstelle (60) um eine Radiofrequenzschnittstelle, eine induktive Schnittstelle oder eine Infrarotschnittstelle handelt.

19. Ohrstöpsel gemäß Anspruch 18, wobei es sich bei der Schnittstelle (60) um eine Bluetooth-Schnittstelle handelt.

20. Ohrstöpsel gemäß Anspruch 1 oder 16, wobei die aktive Einheit (12) Dosimetermittel (88) zum Bestimmen der Schallexposition des Benutzers mittels des Mikrofons (86) aufweist.

21. Ohrstöpsel gemäß Anspruch 20, wobei die Dosimetermittel (88) ausgebildet sind, um die erfasste Schallexposition mit in den Dosimetermitteln implementierten Regeln zu vergleichen, um zu entscheiden, ob die Schallexposition den Regeln entspricht.

22. Ohrstöpsel gemäß einem der vorhergehenden Ansprüche, wobei die Schale (10) eine Außenfläche aufweist, die gemäß der gemessenen Innenform des Außenohrs und Gehörgangs des Benutzers individuell geformt ist.

23. Ohrstöpsel gemäß Anspruch 22, wobei die Schale (10) eine Elastizität zwischen Shore D85 und Shore D65 aufweist.

24. Ohrstöpsel gemäß Anspruch 23, wobei die Schale (10) aus Polyamid gefertigt ist.

25. Ohrstöpsel gemäß Anspruch 18, wobei es sich bei der Schnittstelle (60) um eine frequenzmodulierte Radiosignalschnittstelle handelt.

26. Verwendung eines Gehörschutzohrstöpsels gemäß einem der vorhergehenden Ansprüche, wobei:
die aktive Einheit (12) mit der Schale (10) verbunden wird, indem die Verbindungsmittel (34, 36, 38, 40, 56, 58, 70, 72, 74, 80, 82, 84) in Eingriff gebracht werden, der Ohrstöpsel mindestens zum Teil in den Gehörgang des Benutzers eingesetzt wird, der Ohrstöpsel aus dem Gehörgang des Benutzers entfernt wird, die aktive Einheit von der Schale getrennt wird, indem die Verbindungsmittel außer Eingriff gebracht werden, und der Ohrstöpsel mindestens zum Teil in den Gehörgang des Benutzers eingesetzt wird.

27. Verwendung gemäß Anspruch 26, wobei ferner der Ohrstöpsel aus dem Gehörgang des Benutzers entfernt wird, eine weitere aktive Einheit (12), die sich funktional von der aktiven Einheit (12) unterscheidet, mit der Schale verbunden wird, indem die Verbindungsmittel (34, 36, 38, 40, 56, 58, 70, 72, 74, 80, 82, 84) in Eingriff gebracht werden, und der Ohrstöpsel mindestens zum Teil in den Gehörgang des Benutzers eingesetzt wird.

28. Gehörschutzsystem mit einem Ohrstöpsel gemäß einem der Ansprüche 1 bis 25, sowie mit einer Mehrzahl von funktional unterschiedlichen aktiven Einheiten (12) mit einem akustischen Ausgangswandler (26) und Mitteln (30, 32, 60) zu Erzeugen von Ausgangs-Audiosignalen für den Ausgangswandler und/oder ein Mikrofon (86), wobei die Verbindungsmittel (34, 36, 38, 40, 56, 58, 70, 72, 74, 80, 82, 84) ausgebildet sind, um eine ausgewählte der aktiven Einheiten mit der Schale lösbar zu verbinden, und wobei Ventilmittel (20, 120, 220, 320) beim Verbinden der ausgewählten der aktiven Einheiten mit der Schale aus einer geschlossenen Position, in welcher die Ventilmittel den Schalldurchlass akustisch verschließen, in eine offene Stellung, in welcher die Ventilmittel den Schalldurchlass akustisch öffnen, verlagerbar sind, wobei die Ventilmittel zu der geschlossenen Stellung hin vorgespannt sind, um den Schalldurchlass akustisch zu schließen, wenn die ausgewählte der aktiven Einheiten von der Schale entfernt wird, und wobei der Ausgangswandler und/oder das Mikrofon über den Schalldurchlass akustisch mit der Schallauslassöffnung verbunden ist, wenn die ausgewählte der aktiven Einheiten mit der Schale verbunden ist.

29. Verfahren zum Herstellen eines Gehörschutzohrstöpsels, wobei im Zuge des Verfahrens:
eine Schale bereitgestellt wird, die mindestens zum Teil im Gehörgang eines Benutzers zu tragen ist und ausgebildet ist, um für eine mechanische akustische Dämpfung von mindestens 10 dB, gemittelt über den hörbaren Frequenzbereich, zu sorgen, wenn sie von dem Benutzer getragen wird, wobei die Schale einen Schalldurchlass (16, 16') aufweist, der sich zwischen einer äußeren Öffnung (14) der Schale und einer inneren Schallauslassöffnung (18, 18') am distalen Ende der Schale erstreckt; und
eine aktive Einheit (12) bereitgestellt wird, die einen akustischen Ausgangswandler (26) und Mittel (30, 32, 60) zum Erzeugen von Ausgangs-Audiosignalen für den Ausgangswandler und/oder ein Mikrofon (86) aufweist und ausgebildet ist, um mit der Schale lösbar verbunden zu werden;
wobei die Schale Ventilmittel (20, 120, 220, 320) aufweist, die beim Verbinden der aktiven Einheit mit der Schale aus einer geschlossenen Stellung, in welcher die Ventilmittel den Schalldurchlass akustisch verschließen, in eine offene Stellung, in welcher die Ventilmittel den Schalldurchlass akustisch öffnen, verlagerbar sind, wobei die Ventilmittel zu der geschlossenen Stellung hin vorgespannt sind, um den Schalldurchlass akustisch zu verschließen, wenn die aktive Einheit von der Schale entfernt wird, und wobei der Ausgangswandler und/oder das Mikrofon über den Schalldurchlass akustisch mit der Schallauslassöffnung akustisch verbunden sind, wenn die aktive Einheit mit der schale verbunden ist;
wobei die Schale einschließlich der Ventilmittel mittels eines additiven schichtweisen Aufbauprozesses hergestellt werden.

## Revendications

1. Bouchon d'oreille de protection auditive comprenant une coque (10) destinée à être portée au moins en partie dans un canal auditif d'un utilisateur, ladite coque étant adaptée pour produire une atténuation acoustique mécanique d'au moins 10 dB en moyenne sur la plage des fréquences audibles lorsqu'elle est portée par l'utilisateur, une unité active (12) comprenant un transducteur de sortie acoustique (26) et des moyens (30, 32, 60) pour produire des signaux audio de sortie pour ledit transducteur de sortie et/ou comprenant un microphone (86), et des moyens (34, 36, 38, 40, 56, 58, 80, 82, 84) pour relier de façon détachable ladite unité active à ladite coque, ladite coque comprenant un passage de son (16, 16') s'étendant entre une ouverture extérieure (14) de ladite coque et une ouverture de sortie de son intérieure (18, 18') à l'extrémité distale de ladite coque, **caractérisé en ce que** ladite coque comprend des moyens formant soupape (20, 120, 320) qui sont mobiles, lors du raccordement de ladite unité active à ladite coque, d'une position fermée dans laquelle lesdits moyens formant soupape ferment acoustiquement ledit passage de son à une position ouverte dans laquelle lesdits moyens formant soupape ouvrent acoustiquement ledit passage de son, lesdits moyens formant soupape étant sollicités vers ladite position fermée afin de fermer acoustiquement ledit passage de son lorsque ladite unité active est retirée de ladite coque, et ledit transducteur de sortie et/ou ledit microphone étant reliés acoustiquement à ladite ouverture de sortie de son par l'intermédiaire dudit passage de son lorsque ladite unité active est reliée à ladite coque.

2. Bouchon d'oreille selon la revendication 1, dans lequel lesdits moyens de liaison (34, 36, 38, 40, 56, 58, 70, 72, 74, 80, 82, 84) comprennent des moyens de scellement (34, 36) pour sceller hermétiquement l'extrémité extérieure dudit passage de son (16, 16') vis-à-vis de l'environnement autour dudit utilisateur par le fait de venir en prise avec ladite unité active (12).

3. Bouchon d'oreille selon l'une des revendications 1 et 2, dans lequel lesdits moyens de liaison (34, 36, 38, 40, 56, 58, 70, 72, 74, 80, 82, 84) sont adaptés pour agrafer, visser ou accoupler par une baïonnette ladite unité active (12) à ladite coque (10).

4. Bouchon d'oreille selon l'une des revendications 1 et 2, dans lequel lesdits moyens de liaison (34, 36, 70, 72, 74, 80, 82, 84) sont adaptés pour agrafer ladite unité active (12) à ladite coque (10), et dans lequel l'une parmi ladite unité active et ladite coque comprend un élément radialement mobile (82) ou élastique (72) adapté pour venir en prise avec un élément d'accouplement (70, 80) disposé sur l'autre parmi ladite unité active et ladite coque.

5. Bouchon d'oreille selon la revendication 4, dans lequel ledit élément radialement mobile (82) est sollicité radialement vers le centre de ladite coque (10).

6. Bouchon d'oreille selon l'une des revendications précédentes, dans lequel ladite coque (10) comprend une cavité extérieure (14) dans laquelle ladite unité active (12) doit être insérée.

7. Bouchon d'oreille selon l'une des revendications précédentes, dans lequel lesdits moyens formant soupape (20, 120, 220, 320) sont adaptés pour produire, dans ladite position fermée, une atténuation acoustique d'au moins 10 dB en moyenne sur la plage des fréquences audibles.

8. Bouchon d'oreille selon l'une des revendications précédentes, dans lequel lesdits moyens formant soupape comprennent un couvercle (20) qui est adapté pour être incliné, lors du raccordement de ladite unité active (12) à ladite coque (10), de ladite position fermée dans laquelle ledit couvercle s'étend sur la totalité de la section transversale dudit passage de son (16) à ladite position ouverte dans laquelle ledit couvercle expose ledit passage de son.

9. Bouchon d'oreille selon l'une des revendications précédentes, dans lequel ladite unité active (12) comprend une saillie (28) pour déplacer lesdits moyens formant soupape (20, 120, 220, 320), lors du raccordement de ladite unité active à ladite coque (10), de ladite position fermée à ladite position ouverte.

10. Bouchon d'oreille selon la revendication 9, dans lequel lesdits moyens formant soupape comprennent un élément de soupape (120, 220, 320) qui est mobile axialement à l'intérieur dudit passage de son (16) à l'aide de ladite saillie (28) entre une première position axiale dans laquelle ledit élément de soupape ferme acoustiquement ledit passage de son et une deuxième position dans laquelle ledit élément de soupape ouvre acoustiquement ledit passage de son.

11. Bouchon d'oreille selon la revendication 10, dans lequel ledit élément de soupape (120, 220, 320) est sollicité vers ladite première position axiale par un élément de ressort (52) disposé au moins en partie à l'intérieur dudit passage de son (16).

12. Bouchon d'oreille selon la revendication 11, dans lequel ledit élément de ressort (52) est agencé de façon distale par rapport audit élément de soupape (120, 220, 320).

13. Bouchon d'oreille selon l'une des revendications 10 à 12, dans lequel ledit élément de soupape (120, 220) repose dans ladite première position axiale contre une surface (52) dudit passage de son (16) pour fermer acoustiquement ledit passage de son.

14. Bouchon d'oreille selon l'une des revendications 10 à 13, dans lequel ledit élément de soupape est une bille (120) ou une plaque (220).

15. Bouchon d'oreille selon l'une des revendications 10 à 14, dans lequel ledit passage de son (16, 16') comprend une partie (16') qui est en angle par rapport à la direction axiale de ladite coque (10) et qui est disposée de façon distale par rapport à ladite première position dudit élément de soupape (120, 220, 320).

16. Bouchon d'oreille selon la revendication 1, dans lequel lesdits moyens pour produire des signaux de sortie audio comprennent un microphone (32) pour convertir un son ambiant en signaux audio d'entrée et une unité de traitement du signal audio (30) pour traiter lesdits signaux audio d'entrée en lesdits signaux audio de sortie.

17. Bouchon d'oreille selon la revendication 1, dans lequel lesdits moyens pour produire des signaux de sortie audio comprennent une interface (60) pour une connexion sans fil avec une source de signal audio distante, telle qu'un microphone distant (64).

18. Bouchon d'oreille selon la revendication 17, dans lequel ladite interface (60) est une interface à fréquence radio, une interface inductive ou une interface à infrarouges.

19. Bouchon d'oreille selon la revendication 18, dans lequel ladite interface (60) est une interface Bluetooth.

20. Bouchon d'oreille selon l'une des revendications 1 ou 16, dans lequel ladite unité active (12) comprend des moyens formant dosimètre (88) pour déterminer, à l'aide dudit microphone (86), l'exposition au son dudit utilisateur.

21. Bouchon d'oreille selon la revendication 20, dans lequel lesdits moyens formant dosimètre (88) sont adaptés pour comparer ladite exposition au son déterminée à des réglementations incorporées dans lesdits moyens formant dosimètre afin de juger si ladite exposition au son est conforme auxdites réglementations.

22. Bouchon d'oreille selon l'une des revendications précédentes, dans lequel ladite coque (10) a une surface extérieure individuellement conformée en fonction de la forme intérieure mesurée de l'oreille externe et du canal auditif de l'utilisateur.

23. Bouchon d'oreille selon la revendication 22, dans lequel ladite coque (10) a une élasticité comprise entre Shore D85 et Shore D65.

24. Bouchon d'oreille selon la revendication 23, dans lequel ladite coque (10) est réalisée en polyamide.

25. Bouchon d'oreille selon la revendication 18, dans lequel ladite interface (60) est une interface de signal radio à modulation de fréquence.

26. Utilisation d'un bouchon d'oreille de protection auditive selon l'une des revendications précédentes, comprenant :
le raccordement de ladite unité active (12) à ladite coque (10) par mise en prise desdits moyens de liaison (34, 36, 38, 40, 56, 58, 70, 72, 74, 80, 82, 84), l'insertion dudit bouchon d'oreille au moins en partie dans ledit canal auditif de l'utilisateur, le retrait dudit bouchon d'oreille dudit canal auditif de l'utilisateur, la déconnexion de ladite unité active et de ladite coque par retrait de la prise desdits moyens de liaison, et l'insertion dudit bouchon d'oreille au moins en partie dans ledit canal auditif de l'utilisateur.

27. Utilisation selon la revendication 26, comprenant de plus : le retrait dudit bouchon d'oreille dudit canal auditif de l'utilisateur, le raccordement d'une autre unité active (12) qui est fonctionnellement différente de ladite unité active (12) à ladite coque (10) par mise en prise desdits moyens de liaison (34, 36, 38, 40, 56, 58, 70, 72, 74, 80, 82, 84), et l'insertion dudit bouchon d'oreille au moins en partie dans ledit canal auditif de l'utilisateur.

28. Système de protection auditive comprenant un bouchon d'oreille selon l'une des revendications 1 à 25, comprenant de plus une pluralité d'unités actives fonctionnellement différentes (12) comprenant un transducteur de sortie acoustique (26) et des moyens (30, 32, 60) pour produire des signaux audio de sortie pour ledit transducteur de sortie et/ou un microphone (86), dans lequel lesdits moyens de liaison (34, 36, 38, 40, 56, 58, 70, 72, 74, 80, 82, 84) sont adaptés pour relier de façon détachable une unité sélectionnée parmi lesdites unités actives à ladite coque, et dans lequel des moyens formant soupape (20, 120, 220, 320) peuvent être déplacés, lors du raccordement de ladite unité sélectionnée parmi lesdites unités actives à ladite coque, d'une position fermée dans laquelle lesdits moyens formant soupape ferment acoustiquement ledit passage de son à une position ouverte dans laquelle lesdits moyens formant soupape ouvrent acoustiquement ledit passage de son, lesdits moyens formant soupape étant sollicités vers ladite position fermée afin de fermer acoustiquement ledit passage de son lorsque ladite unité sélectionnée parmi lesdites unités actives est retirée de ladite coque, et dans lequel ledit transducteur de sortie et/ou ledit microphone sont acoustiquement reliés à ladite ouverture de sortie de son par l'intermédiaire dudit passage de son lorsque ladite unité sélectionnée parmi lesdites unités actives est reliée à ladite coque.

29. Procédé pour fabriquer un bouchon d'oreille de protection auditive, comprenant :
la réalisation d'une coque (10) destinée à être portée au moins en partie dans un canal auditif d'un utilisateur, ladite coque étant adaptée pour produire une atténuation acoustique mécanique d'au moins 10 dB en moyenne sur la plage des fréquences audibles lorsqu'elle est portée par l'utilisateur, et ladite coque comprenant un passage de son (16, 16') s'étendant entre une ouverture extérieure (14) de ladite coque et une ouverture de sortie de son intérieure (18, 18') à l'extrémité distale de ladite coque ; et
la réalisation d'une unité active (12) comprenant un transducteur de sortie acoustique (26) et des moyens (30, 32, 60) pour produire des signaux audio de sortie pour ledit transducteur de sortie et/ou un microphone (86), ladite unité active étant adaptée pour être reliée de façon détachable à ladite coque ;
ladite coque comprenant des moyens formant soupape (20, 120, 220, 320) qui sont mobiles, lors du raccordement de ladite unité active à ladite coque, d'une position fermée dans laquelle lesdits moyens formant soupape ferment acoustiquement ledit passage de son à une position ouverte dans laquelle lesdits moyens formant soupape ouvrent acoustiquement ledit passage de son, lesdits moyens formant soupape étant sollicités vers ladite position fermée afin de fermer acoustiquement ledit passage de son lorsque ladite unité active est retirée de ladite coque, et ledit transducteur de sortie et/ou ledit microphone étant reliés acoustiquement à ladite ouverture de sortie de son par l'intermédiaire dudit passage de son lorsque ladite unité active est reliée à ladite coque ;
dans lequel ladite coque comprenant lesdits moyens formant soupape est fabriquée à l'aide d'un procédé d'accumulation couche sur couche additif.
